(19) Europäisches Patentamt / European Patent Office / Office européen des brevets

(11) **EP 3 390 593 B1**

(12) **FASCICULE DE BREVET EUROPEEN**

(45) Date de publication et mention de la délivrance du brevet:
**30.10.2019 Bulletin 2019/44**

(21) Numéro de dépôt: **16822123.2**

(22) Date de dépôt: **13.12.2016**

(51) Int Cl.:
*C10M 137/04* (2006.01)    *C10M 173/00* (2006.01)

(86) Numéro de dépôt international:
**PCT/EP2016/080795**

(87) Numéro de publication internationale:
**WO 2017/102726 (22.06.2017 Gazette 2017/25)**

(54) **ESTERS DE PHOSPHATE ALCOXYLÉS POUR COMPOSITIONS LUBRIFIANTES**

PHOSPHORSÄUREESTER VON ALKOXYLIERTEN ALKOHOLEN ZUR VERWENDUNG IN SCHMIERSTOFFEN

PHOSPHORIC ACID ESTER OF ALKOXYLATED ALCOHOLS FOR LUBRICANT COMPOSITIONS

(84) Etats contractants désignés:
AL AT BE BG CH CY CZ DE DK EE ES FI FR GB GR HR HU IE IS IT LI LT LU LV MC MK MT NL NO PL PT RO RS SE SI SK SM TR

(30) Priorité: **14.12.2015 FR 1562264**

(43) Date de publication de la demande:
**24.10.2018 Bulletin 2018/43**

(73) Titulaire: **Rhodia Operations**
**75009 Paris (FR)**

(72) Inventeurs:
• **HEDOIRE, Claude-Emmanuel**
**95460 Ezanville (FR)**

• **GIRONDA, Ramona**
**20021 Ospiate Di Bollate (IT)**

(74) Mandataire: **Cordier, Pascal Christian et al**
**Rhodia Operations**
**Direction de la Propriété Industrielle**
**40, rue de la Haie Coq**
**93306 Aubervilliers (FR)**

(56) Documents cités:
WO-A1-01/88070     DE-A1- 2 756 747
US-A- 3 933 658     US-A- 4 613 445

**Description**

La présente invention a trait à des additifs pour des compositions lubrifiantes destinées à la mise en forme et au travail des métaux, et plus particulièrement les compositions utilisées lors des opérations de découpe de métaux, qui se présentent typiquement sous forme d'émulsions, typiquement d'huiles naphténiques ou paraffiniques, dans l'eau. L'invention concerne plus spécifiquement des agents émulsifiants capables de fournir, entre autres, un effet lubrifiant additionnel.

Des agents émulsifiants fournissant un effet lubrifiant de ce type sont déjà connus, et notamment pour des applications dans le travail de métaux, en particulier pour la découpe de métaux. Entre autres, il a été proposé dans ce cadre l'emploi d'émulsifiant de type esters de phosphates, qui présentent l'avantage de fournir des effets intéressant en plus de leur caractère émulsifiant, et notamment un effet de lubrification. Cet effet de lubrification, plus ou moins prononcé selon la nature exacte de l'ester de phosphate, semble s'expliquer au moins en partie par une adsorption des groupes phosphorés sur la surface des métaux, ce par quoi les esters de phosphate forment, schématiquement, une sorte de couche protectrice anti-usure à la surface des métaux. Les esters de phosphate fournissent en outre, pour la plupart, un effet de protection spécifique de l'aluminium dont ils évitent le noircissement par corrosion ("*staining*"). Par ailleurs, les esters de phosphate sont pour la plupart biodégradables.

Cela étant, en marge des avantages précités, les émulsifiants à caractère lubrifiant connus jusqu'alors présentent des inconvénients. En particulier, ils induisent pour la plupart une formation non négligeable de mousse, qui limite, voire interdit, leur emploi dans certaines applications. De plus, ils tendent à précipiter en formant des savons lorsqu'ils sont employés en eau dure. En outre, certains esters de phosphate sont écotoxiques. A ce jour, il n'a pas été décrit d'émulsifiant efficace ne présentant pas un de ces inconvénients. L'émulsifiant Lubrhophos® LB400, par exemple, disponible auprès de la société Solvay, fournit un effet lubrifiant (anti-usure) marqué, mais il forme des savons en eau dure. Des émulsifiants présentant une tendance réduite à la formation de savons ont été décrits, comme le Lubrhophos® RD510E par exemple, mais ils présentent en général des propriétés médiocres en lubrification.

Un but de la présente invention est de fournir des émulsifiants à caractère lubrifiant utilisables dans des compositions pour le travail des métaux qui présentent un bon effet lubrifiant (anti-usure) et qui ne présentent pas les inconvénients précités.

A cet effet, la présente invention propose des esters de phosphate alcoxylés spécifiques, dont les inventeurs ont maintenant mis en évidence que, de façon inattendue, ils présentent de très bonnes propriétés de lubrification conjointement avec une faible tendance à la formation de mousse, une formation très réduite de savon en eaux dures et en outre une faible écotoxicité.

Selon un premier aspect, la présente invention concerne ces esters de phosphate alcoxylés particuliers. Plus précisément, l'invention a pour objet une composition polymère incluant des polymères répondant à la formule (I) ci-dessous :

$$[R\text{-}O(\text{-}CH(CH_3)\text{-}CH_2O\text{-})_n(CH_2\text{-}CH_2O\text{-})_p\text{-}]_{1+x}P(=O)(OPH)_{2-x} \qquad (I)$$

où :

R est un groupe hydrocarboné, saturé ou insaturé, linéaire ou ramifié, et de préférence linéaire, comprenant de 8 à 12 atomes de carbone

n est un nombre, entier ou non, compris entre 6 et 20

p est un nombre, entier ou non, compris entre 4 et 25

x est un nombre compris entre 0 et 1, notamment entre 0,1 et 0,9

La formule (I) représente la composition moyenne en nombre de la population de polymères.

Les polymères présents dans cette composition comprennent en général un mélange de monoesters de formule :

$$R\text{-}O(\text{-}CH(CH_3)\text{-}CH_2O\text{-})_n(CH_2\text{-}CH_2O\text{-})_p\text{-}P(=O)(OH)_2$$

et de diesters de formule :

$$[R\text{-}O(\text{-}CH(CH_3)\text{-}CH_2O\text{-})_n(CH_2\text{-}CH_2O\text{-})_p\text{-}]_2P(=O)OH$$

x reflète le rapport molaire entre diesters et monoesters (x=0 correspond au cas où la composition comporte uniquement des monoesters ; x=1 correspond au cas où la composition comporte uniquement des diesters).

La population de polymères peut comprendre plusieurs types de polymères comprenant des groupes R distincts.

Dans la formule (I), le groupe R est de préférence un groupe alkyle saturé ou monoinsaturé. Il s'agit avantageusement d'un alkyle linéaire. Le groupe R est un groupe en C8 à C12, à savoir qu'il contient au moins 8 et au plus 12 atomes de carbone. Avantageusement, il s'agit d'un groupe en C11 à C12. Selon un mode de réalisation particulièrement intéressant, le groupe R contient exactement 12 atomes de carbone (groupe en C12).

Un groupement R particulièrement indiqué pour l'invention est le groupe lauryl (alkyl en C12 linéaire et saturé). Les polymères de l'invention répondent alors à la formule (Ia) suivante :

$$[CH_3\text{-}(CH_2)_{11}\text{-}O\text{-}(CH(CH_3)\text{-}CH_2O\text{-})_n(\text{-}CH_2\text{-}CH_2O\text{-})_p]_{1+x}\text{-}P(=O)(OH)_{2-x} \qquad (Ia)$$

où n, p et x ont les significations précitées, x étant en général entre 0,1 et 0.9.

En pratique, les polymères de formule (Ia) sont obtenus au départ d'un mélange d'alcools de la coupe laurique et ils peuvent donc être présent dans la composition de l'invention en mélange avec d'autres polymères qui contiennent typiquement des groupes R de type alkyles en C8 à C16.

Les composés de formule (I) et (Ia) précités comportent, dans cet ordre, entre le groupe R et le groupe -P(=O)(OH)$_2$, d'abord un enchaînement d'unités oxyde de propylène puis un enchaînement d'unités oxyde d'éthylène.

Dans les formules ci-dessus, n représente le nombre d'unités oxyde de propylène si on se réfère à un composé isolé (auquel cas n est nécessairement entier) ou bien, le plus souvent, au nombre moyen d'unités oxyde de propylène dans le cas d'une population comprenant un mélange de composés (auquel cas n peut être entier ou non). De même, p représente le nombre d'unités oxyde d'éthylène si on se réfère à un composé isolé (auquel cas p est nécessairement entier) ou bien, le plus souvent, au nombre moyen d'unités oxyde d'éthylène dans le cas d'une population comprenant un mélange de composés (auquel cas p peut être entier ou non).

Dans les composés de l'invention, n est inférieur ou égal à 20 et reste en général inférieur ou égal à 15, et typiquement inférieur ou égal à 10. Par ailleurs n est supérieur ou égal à 6 et plus préférentiellement supérieur ou égal à 6,5, par exemple entre 7 et 10.

Par ailleurs, dans les composés de l'invention, p est inférieur ou égal à 25 et reste en général inférieur ou égal à 20, et typiquement inférieur ou égal à 15. Par ailleurs p est supérieur ou égal à 4 et plus préférentiellement supérieur ou égal à 4,5, par exemple entre 4,5 et 10.

Selon un mode de réalisation intéressant, n est égal à 7,5 et p est égal à 5.

Un composé particulièrement utile selon l'invention répond à la formule (Ib) suivante :

$$[CH_3\text{-}(CH_2)_{11}\text{-}O\text{-}(CH(CH_3)\text{-}CH_2O)_{7,5}(\text{-}CH_2\text{-}CH_2O\text{-})_5]_{1+x}\text{-}P(=O)(OH)_{2-x} \qquad (Ib)$$

où x a la signification précitée, x étant en général entre 0,1 et 0.9.

Ces polymères de formule (Ib), qui sont un cas particulier des polymères (Ia) précités sont eux aussi typiquement obtenus au départ d'un mélange d'alcools de la coupe laurique et peuvent donc être présents dans la composition de l'invention en mélange avec d'autres polymères qui contiennent typiquement des groupes R de type alkyles en C8 à C16.

Les polymères de l'invention peuvent être aisément synthétisés. Typiquement par propoxylation d'un alcool ROH (ou d'un mélange d'alcools) où R est le groupe présent dans le composé à synthétiser, ce par quoi on forme un composé RO-(CH(CH$_3$)-CH$_2$O)$_n$H qu'on éthoxyle ensuite pour former un composé RO-(CH(CH$_3$)-CH$_2$O)$_n$(CH$_2$-CH$_2$O)$_p$H qui peut être converti en l'ester de phosphate de formule (I) par tout moyen connu en soi, par exemple par réaction avec P$_2$O$_5$, notamment dans les conditions de l'étape 2 de l'exemple 1 ci-après.

Selon un autre aspect, l'invention concerne l'utilisation des compositions incluant des polymères répondant à l'une des formules (I), (Ia) ou (Ib) précitées dans une formulation lubrifiante pour le travail ou la mise en forme des métaux, de préférence une émulsion lubrifiante, ladite composition étant typiquement une composition lubrifiante pour le découpage de métaux, en particulier d'aluminium.

Les compositions de l'invention fournissent dans ce cadre un effet de lubrification, en formant schématiquement à la surface des métaux une couche protectrice anti-usure. Sur l'aluminium, ils inhibent en outre le noircissement (coloration) pas oxydation (« staining »),

De plus, les compositions de l'invention présentent des propriétés émulsifiantes et elles sont donc propres à stabiliser une émulsion. Elles peuvent de ce fait être employés à la fois pour stabiliser une émulsion lubrifiante et pour améliorer les qualités émulsifiante de ladite émulsion. Typiquement, pour former une émulsion de ce type, les compositions de l'invention peuvent être incorporées dans une huile (typiquement à hauteur de 5 à 30%, par exemple entre 10 et 25% , notamment environ 20%, par rapport à la masse totale composé + huile), puis ce mélange est mélangé à de l'eau (typiquement de l'ordre de 5 à 15% de mélange dans 85 à 95% d'eau, ces pourcentages étant en masse par rapport à la masse totale du mélange eau+composé+huile).

Les compositions de l'invention, et tout particulièrement celles comprenant des polymères de formule (Ia) et (Ib), présentent l'avantage d'être peu moussants. Ils sont en cela aussi intéressant que les agents du type du Lubrhophos® LB400 et beaucoup plus intéressants que le Lubrhophos® RD510E.

Par ailleurs, les composés de l'invention, et tout particulièrement les composés de formule (Ia) et (Ib), ont une tendance réduite à la précipitation par formation de savons en eaux dures. De plus, ils ont une ecotoxicité très faible, inférieure notamment à celle du Lubrhophos® LB400. Ainsi, les compositions de l'invention constitue une alternative très intéressante au Lubrhophos® LB400.

Différents aspects et avantages de l'invention seront encore illustrés par les exemples ci-après.

# EXEMPLES

## Exemple 1

## *Composition comprenant un polymère de formule (Ib)*

## Etape 1 : alkoxylation (propoxylation puis éthoxylation)

On a utilisé les réactifs suivants, dans les proportions indiqués en pourcentages massique par rapport à la masse totale de réactifs :

| | |
|---|---|
| Alcool laurique $CH_3$-$(CH_2)_{11}$-OH | : 21, 4% |
| Hydroxyde de potassium | : 0,1% |
| Oxyde de propylene | : 47,3% |
| Oxyde d'éthylene | : 31,1% |
| Acide acétique | : 0,1 % |

L'acide laurique et l'hydroxyde de potassium ont été chargés dans un réacteur équipé d'un agitateur interne à boucle de recirculation. Ils ont été déshydratés sous vide à 130°C pendant 30 minutes (humidité finale < 0,030%).

L'oxyde de propylene a alors été introduit lentement, en refroidissant (reaction exothermique) afin de maintenir la température à 130°C +/-2°C.

A l'issue de cette propoxylation, le milieu réactionnel a été maintenu à la température de 130°C +/-2°C jusqu'à obtention d'une pression constante dans le réacteur, indiquant la fin de la consommation de l'oxyde de propylène On a alors introduit lentement l'oxyde d'éthylène, là encore en refroidissant du fait de exothermie pour maintenir la température à 130°C +/-2°C.

A l'issue de cette éthoxylation, le milieu réactionnel a été maintenu à la température de 130°C +/-2°C jusqu'à obtention d'une pression constante dans le réacteur, indiquant la fin de la consommation de l'oxyde d'ethylene. On a alors refroidi le milieu à 50°C et on a neutralize par ajout de l'acide acétique.

## Etape 2 : formation de l'ester de phosphate

On a utilisé les réactifs suivants, dans les proportions indiqués en pourcentages massique par rapport à la masse totale de réactifs :

| | |
|---|---|
| alcoxylate d'alcool laurique formé dans l'étape 1 | : 94,25 % |
| H3PO2 solution aqueuse 50% | : 0,2% |
| P2O5 | : 4,85% |
| Eau | : 0,5% |
| Peroxyde d'hydrogene | : 0,2% |

Dans un réacteur muni d'un agitateur magnétique et sous atmosphère d'azote, l'alcoxylate d'alcool laurique formé dans l'étape 1 a été chargé à 40°C, et on a immédiatement introduit la solution aqueuse de $H_3PO_2$, de façon à éviter une oxydation intempestive en fin de réaction.

On a ajouté le $P_2O_5$ progressivement, sur une durée de 5 heures, en maintenant la température à 60°C. Après la fin de l'addition de P2O5, on a laissé la réaction se poursuivre à 80 °C pendant deux heures.

A l'issue de ces deux heures, on a ajouté l'eau et on a laissé le milieu refroidir jusqu'à 60°C. Une fois cette température atteinte, on a ajouté la solution de peroxyde d'hydrogène.

*Exemple 2*

*Propriétés des compositions de polymères de l'exemple 1*

La composition de polymère synthétisée dans l'exemple précédent a été utilisée pour la préparation d'émulsions dans les conditions ci-dessous :

La composition telle qu'obtenue à l'issue de l'exemple 1 (qui est un concentrat de polymères) a été diluée dans de l'eau désionisée avec un ratio massique concentrat/eau ajouté de 10/90 dans une éprouvette graduée de 100 mL, en tamponnant le pH à 9 (solution de MEA). On a ensuite mélangé le milieu aqueux tamponné à une huile naphténique (Nytex 810 de la société Nynas), avec un rapport massique milieu aqueux/huile de 20/80, ce par quoi on a formé une première émulsion E1.

En parallèle, on a réalisé une seconde émulsion E2 dans les mêmes conditions, mais en substituant l'eau desionisée par une eau dure comprenant 347mg/L de $CaCO_3$ et 41,1mg/L de $MgCO_3$.

**Propriétés émulsifiantes**

La stabilité à court terme (au bout de 30 minutes) et long terme (au bout de 7 jours, à 40°C) l'émulsion des émulsions E1 et E2 a été évaluée comme suit :

Selon la stabilité d'une émulsion, plusieurs phases peuvent apparaître en plus de la phase constituant l'émulsion : une phase crémeuse et/ou une phase huileuse (généralement surnageant) et/ou une phase aqueuse (généralement sous l'émulsion). La stabilité peut être chiffrée en mesurant les volumes respectifs des différentes phases, et on définit un index de stabilité d'émulsion $I_{stabilité}$ comme suit :

$$I_{stabilité} = 100 - \text{volume de la phase crèmeuse (exprimé en mL)} - 5 \times \text{volume de la phase huileuse d'huile (exprimé en mL)} - 5 \times \text{volume de la phase aqueuse (exprimé en mL)}$$

L'émulsion est d'autant plus stable que son indice de stabilité est élevé : L'émulsion est dite :

« très stable » à partir de de 95
« acceptable » entre 80 et 95
« peu stable » en dessous de 80 et jusqu'à 60
« instable » en dessous de 60

Les dispersions E1 et E2 sont de type très stable, avec des indices de stabilité d'au minimum 95, reportés dans le Tableau 1 ci-dessous :

**Tableau 1** : stabilité des émulsions à court et long terme

| Emulsion | $I_{stabilité}$ après 30 minutes | $I_{stabilité}$ après 7 jours à 40°C |
|----------|----------------------------------|--------------------------------------|
| E1 | 95 | 98 |
| E2 | 96 | 97 |

**Formation de mousse et/ou de savon**

La tendance au moussage a été évaluée selon un test employant une pompe centrifuge et une éprouvette graduée à chemise d'eau de 2 L munie d'une sortie latérale proche du fond de l'éprouvette. Les émulsions ont été introduites dans le cylindre jusqu'à la graduation 1000 mL, et elles ont été pompées via la sortie au fond de l'éprouvette avec un débit de 250L/h, et réinjectées via la pompe dans le cylindre, d'une hauteur de 390 mm au dessus de la graduation 1000 mL. On a effectué le pompage en vue d'obtenir une formation de mousse atteignant la graduation 2000mL, pendant une durée maximale $t_{MAX}$ de 5 heures :

Dans le cas où la graduation 2000mL a été atteinte avant 5 heures : on a noté le temps ($t_{2000} < t_{MAX}$) mis pour atteindre la graduation 2000mL (à savoir un volume de mousse $V_{max}$ correspondant au volume entre les indices 1000mL et 2000 mL) et on a immédiatement arrêté le pompage à $t_{2000}$, puis on a mesuré le volume ($V_{t2000+15min}$)

de mousse au-dessus de la graduation 1000 mL au temps $t_{2000m}$+15 minutes.

<u>Dans le cas où la graduation n'est pas atteinte au bout de cinq heures</u> : on a mesuré le volume de mousse ($V_{5h <} V_{max}$) atteint au-dessus de la graduation 1000 mL au bout de cinq heures ($t_{MAX}$) et on a arrêté la pompe au bout de 5 heures, puis on a mesuré le volume ($V_{5h+15min}$) de mousse au-dessus de la graduation 1000 mL 15 minutes après l'arrêt de la pompe.

On définit pour les deux cas :

un temps de fin de pompage $t_P$
égal à $t_{2000}$ dans le premier cas et à $t_{MAX}$ dans le second
un volume de fin de pompage $V_P$
égal à $V_{MAX}$ dans le premier cas et à $V_{5h}$ dans le second
un volume de mousse après repos de 15 minutes $V_R$
égal à $V_{MAX}$ dans le premier cas et à $V_{5h}$ dans le second

Deux indices reflétant le profil de la mousse sont calculés comme suit :

- **Indice de niveau de mousse initial $I_M$:**

$$I_M = 100 - 10 \times (1+ \log (V_P/ t_P)$$

- **Indice de démoussage $I_D$:**

$$I_D = 100 \times (V_P - V_R)/V_P$$

On a également déterminé visuellement la formation ou non de savon sur les parois.

Les résultats obtenus pour les dispersions E1 et E2 sont reportés dans le Tableau 1 ci-dessous :

**Tableau 2** : profils de moussage des émulsions

| Emulsion | $I_M$ | ID | Formation de savon |
|---|---|---|---|
| E1 | 70 | 85 | Très faible |
| E2 | 75 | 95 | Très faible |

*Propriétés anti-usure*

Elles ont été évaluées à l'aide de la méthode ASTM D2670.

Le tribomètre employé est un Falex muni d'un système *"Pin and Vee blocks"*, immergé dans les émulsions à tester, à une température maintenue à 24 °C (dans un réservoir chemisé d'eau) et à 700 lbs pendant 10 min.

L'usure est déterminée par la perte de poids du système *"Pin and Vee blocks"*, qui reflète le niveau d'usure. On a obtenu une perte de poids très faible de l'ordre de 10 mg pour les deux émulsions, ce qui reflète de très bonnes propriétés lubrifiantes.

*Inhibition de la corrosion (aluminium)*

On a testé les émulsions E1 et E2 sur trois type d'alliage à base d'aluminum, à soir les alliages 2024, 6061, and 7075.

Des éprouvettes constituées par chacun des alliages ont été plongés dans les émulsions dans des bouteilles fermées et maintenues à 40°C dans ces conditions durant quatre semaines.

La corrosion est évaluée visuellement, la surface virant au gris foncé, voire au noir, dans le cas d'une corrosion ("Staining"). Aucune corrosion sensible n'a été détectée au bout de quatre semaines, contrairement à des témoins dans l'eau.

On peut également quantifier la prise de masse des échantillons au bout de 4 semaines, qui reflètent la corrosion : moins de 0,1% en masse avec les émulsions E1 et E2 contre environ 30% en masse avec de l'eau.

**Revendications**

1. Composition polymère incluant des polymères répondant à la formule (I) ci-dessous :

$$[R-O(-CH(CH_3)-CH_2O-)_n(CH_2-CH_2O-)_p-]_{1+x}P(=O)(OH)_{2-x} \qquad (I)$$

où :

R est un groupe hydrocarboné, saturé ou insaturé, linéaire ou ramifié comprenant de 8 à 12 atomes de carbone
n est un nombre, entier ou non, compris entre 6 et 20
p est un nombre, entier ou non, compris entre 4 et 25
x est un nombre compris entre 0 et 1, notamment entre 0,1 et 0,9.

2. Composition selon la revendication 1, où le groupe R est un groupe alkyle saturé ou monoinsaturé, de préférence linéaire.

3. Composition selon la revendication 2, où les polymères répondent à la formule (Ia) suivante :

$$[CH_3-(CH_2)_{11}-O-(CH(CH_3)-CH_2O-)_n(-CH_2-CH_2-O)_p]_{1+x}-P(=O)(OH)_{2-x} \qquad (Ia)$$

où n, p et x sont tels que définis dans la revendication 1.

4. Composition selon l'une des revendications 1 à 3, où n est compris entre 6,5 et 15, par exemple entre 7 et 10.

5. Composition selon l'une des revendications 1 à 3, où p est compris entre 4,5 et 20, par exemple entre 4,5 et 10.

6. Composition selon l'une des revendications 1 à 5, où n est égal à 7,5 et p est égal à 5.

7. Composition selon la revendication 6, où les polymères répondent à la formule (Ib) suivante :

$$[CH_3-(CH_2)_{11}-O-(CH(CH_3)-CH_2O)_{7,5}(-CH_2-CH_2O-)_5]_{1+x}-P(=O)(OH)_{2-x} \qquad (Ib)$$

où x est tel que défini dans la revendication1.

8. Utilisation d'une composition selon l'une des revendications 1 à 7, dans une formulation lubrifiante pour le travail ou la mise en forme des métaux, de préférence une émulsion lubrifiante

9. Utilisation selon la revendication 8, où la formulation est une formulation lubrifiante pour le découpage de métaux.

10. Utilisation selon la revendication 8 ou 9, où la formulation est une formulation lubrifiante pour le travail ou la mise en forme, notamment pour le découpage, d'aluminium.

**Patentansprüche**

1. Polymerzusammensetzung, enthaltend Polymere, die der nachstehenden Formel (I) entsprechen:

$$[R-O(-CH(CH_3)-CH_2O-)_n(CH_2-CH_2O-)_p-]_{1+x}P(=O)(OH)_{2-x} \qquad (I)$$

wobei:

R für eine lineare oder verzweigte, gesättigte oder ungesättigte Kohlenwasserstoffgruppe mit 8 bis 12 Kohlenstoffatomen steht,
n für eine ganze oder gebrochene Zahl zwischen 6 und 20 steht,
p für eine ganze oder gebrochene Zahl zwischen 4 und 25 steht,
x für eine Zahl zwischen 0 und 1, insbesondere zwischen 0,1 und 0,9, steht.

2. Zusammensetzung nach Anspruch 1, wobei die Gruppe R für eine vorzugsweise lineare, gesättigte oder einfach ungesättigte Alkylgruppe steht.

3. Zusammensetzung nach Anspruch 2, wobei die Polymere der folgenden Formel (Ia) entsprechen:

$$[CH_3\text{-}(CH_2)_{11}\text{-}O\text{-}(CH(CH_3)\text{-}CH_2O\text{-})_n(\text{-}CH_2\text{-}CH_2O\text{-})_p]_{1+x}\text{-}P(=O)(OH)_{2-x} \qquad (Ia)$$

wobei n, p und x wie in Anspruch 1 definiert sind.

4. Zusammensetzung nach einem der Ansprüche 1 bis 3, wobei n zwischen 6,5 und 15, beispielsweise zwischen 7 und 10, liegt.

5. Zusammensetzung nach einem der Ansprüche 1 bis 3, wobei p zwischen 4,5 und 20, beispielsweise zwischen 4,5 und 10, liegt.

6. Zusammensetzung nach einem der Ansprüche 1 bis 5, wobei n gleich 7,5 ist und p gleich 5 ist.

7. Zusammensetzung nach Anspruch 6, wobei die Polymere der folgenden Formel (Ib) entsprechen:

$$[CH_3\text{-}(CH_2)_{11}\text{-}O\text{-}(CH(CH_3)\text{-}CH_2O)_{7,5}(\text{-}CH_2\text{-}CH_2O\text{-})_5]_{1+x}\text{-}P(=O)(OH)_{2-x} \qquad (Ib)$$

wobei x wie in Anspruch 1 definiert ist.

8. Verwendung einer Zusammensetzung nach einem der Ansprüche 1 bis 7 in einer Schmiermittelformulierung zur Bearbeitung oder Umformung von Metallen, vorzugsweise einer Schmiermittelemulsion.

9. Verwendung nach Anspruch 8, wobei es sich bei der Formulierung um eine Schmiermittelformulierung zum Schneiden von Metallen handelt.

10. Verwendung nach Anspruch 8 oder 9, wobei sich bei der Formulierung um eine Schmiermittelformulierung zur Bearbeitung oder Umformung, insbesondere zum Schneiden, von Aluminium handelt.


**Claims**

1. Polymer composition including polymers corresponding to the formula (I) below:

$$[R\text{-}O(\text{-}CH(CH_3)\text{-}CH_2O\text{-})_n(CH_2\text{-}CH_2O\text{-})_p\text{-}]_{1+x}\ P(=O)(OH)_{2-x} \qquad (I)$$

where:

R is a linear or branched, saturated or unsaturated hydrocarbon-based group comprising from 8 to 12 carbon atoms
n is an integer or decimal number between 6 and 20
p is an integer or decimal number between 4 and 25
x is a number between 0 and 1, in particular between 0.1 and 0.9.

2. Composition according to Claim 1, where the R group is a preferably linear, saturated or monounsaturated alkyl group.

3. Composition according to Claim 2, where the polymers correspond to the following formula (Ia):

$$[CH_3\text{-}(CH_2)_{11}\text{-}O\text{-}(CH(CH_3)\text{-}CH_2O\text{-})_n(\text{-}CH_2\text{-}CH_2O\text{-})_p]_{1+x}\text{-}P(=O)(OH)_{2-x} \qquad (Ia)$$

where n, p and x are as defined in Claim 1.

4. Composition according to one of Claims 1 to 3, where n is between 6.5 and 15, for example between 7 and 10.

5. Composition according to one of Claims 1 to 3, where p is between 4.5 and 20, for example between 4.5 and 10.

6. Composition according to one of Claims 1 to 5, where n is equal to 7.5 and p is equal to 5.

7. Composition according to Claim 6, where the polymers correspond to the following formula (Ib):

$$[CH_3\text{-}(CH_2)_{11}\text{-}O\text{-}(CH(CH_3)\text{-}CH_2O)_{7,5}(\text{-}CH_2\text{-}CH_2O\text{-})_5]_{1+x}\text{-}P(=O)(OH)_{2-x} \qquad (Ib)$$

where x is as defined in Claim 1.

8. Use of a composition according to one of Claims 1 to 7, in a lubricating formulation for working or shaping metals, preferably a lubricating emulsion.

9. Use according to Claim 8, where the formulation is a lubricating formulation for cutting metals.

10. Use according to Claim 8 or 9, where the formulation is a lubricating formulation for working or shaping, in particular for cutting, aluminium.